# EUROPEAN PATENT SPECIFICATION

(11) **EP 1 146 870 B1**
(45) Date of publication and mention of the grant of the patent: **23.08.2006**
(21) Application number: 00901737.7
(22) Date of filing: 31.01.2000
(51) Int. Cl.: A61K 31/355, A61K 31/375, A23K 1/16, A23K 1/18, A61P 9/00, A61P 13/12, A61P 19/02, A61P 25/00, A61P 27/12, A61P 29/00, A61P 37/00, A61P 39/00

(54) **ANTIOXIDANT COMPOSITIONS FOR COMPANION ANIMALS**
ANTIOXIDATIVE ZUSAMMENSETZUNGEN FÜR HAUSTIERE
COMPOSITIONS ANTIOXYDANTES POUR LES ANIMAUX DE COMPAGNIE

(30) Priority: 29.01.1999 GB 9902051; 02.12.1999 GB 9928549
(43) Date of publication of application: 24.10.2001
(73) Proprietor: MARS UK LIMITED, Slough, Berkshire SL1 4LG (GB)
(72) Inventor: HARPER, Jean E., Waltham Centre for Pet Nutrition, Leicestershire LE14 4RT (GB)
(74) Representative: Cornish, Kristina Victoria Joy
(86) International application number: PCT/GB2000/000270
(87) International publication number: WO 2000/044375

(56) References cited:
- EP-A- 0 015 721
- EP-A- 0 834 262
- EP-A- 0 848 955
- WO-A-00/01399
- WO-A-97/35491
- GB-A- 2 185 673
- US-A- 3 384 545
- US-A- 5 141 755
- DATABASE WPI Section Ch, Week 199723 Derwent Publications Ltd., London, GB; Class B05, AN 1997-259340 XP002137042 & ZA 9 605 149 A (DAVIS H J), 26 March 1997 (1997-03-26)
- K. PRASAD ET AL.: "Oxidative stress as a mechanism of cardiac failure in chronic volume overload in canine model" JOURNAL OF MOLECULAR AND CELLULAR CARDIOLOGY, vol. 28, no. 2, 1996, pages 375-385, XP000909089 ACADEMIC PRESS, LONDON, GB ISSN: 0022-2828
- DATABASE WPI Section Ch, Week 199521 Derwent Publications Ltd., London, GB; Class B07, AN 1995-157784 XP002137041 -& JP 07 079712 A (SANEIGEN FFI KK), 28 March 1995 (1995-03-28)

## Description

Free radicals are inherent in the aerobic metabolism of living organisms and are generated by both physiological and pathological processes. They are sometimes generated intentionally to serve biological functions, such as microbicides in phagocyte cells, or may be accidents of chemistry following which they exhibit destructive behaviours. Whatever their mechanism of generation, if free radical production and removal is not controlled, then their effects on an organism can be damaging. To combat excessive and inappropriate damage, an elaborate system of antioxidant defences has evolved.

When there is an unbalance between the oxidants and the antioxidants in favour of the oxidants, a condition of oxidative stress exists that can lead to tissue damage.

Patent application EP-A-0848955 relates to an anti-stress agent for animals comprising an ascorbic acid derivative. US Patent Application 5141755 discloses a nutritionally balanced foodstuff for pets comprising vitamin E (0.05% weight), vitamin C (0.10% weight) and taurine (0.05% weight), if the foodstuff is for cats, and vitamin A. Document ZA9605149A describes a foodstuff to prevent and treat viral infections in pets comprising vitamin E, vitamin C and a carotenoid. An article in The Journal of Mollecular and Cellullar Cardiology by K Prasad *et al* relates to a foodstuff comprising vitamin E to reduce oxidative stress and reduce cardiac failure. Patent Application WO 00/01399A describes the use of vitamin E against inflammation in pets. Patent Application JP 07079712A relates to a food, colour and taste enhancer comprising vitamin E and a carotenoid. Document GBA 2185673 describes a foodstuff comprising vitamin E and carotenoid. USA 3384545 relates to injectable composition comprising vitamin E and vitamin A. European Patent EP-A-0015721 describes an arthritis treatment comprising vitamin C. EP-A-0834262 relates to an anti-inflammatory, anti-mutagenic, anti-fat and anti-oxidant composition comprising vitamin E. Document WO 97/35491A describes a carotenoid for the use of increasing production in breeding. EP 0695508 describes a cat meal dressing that can be added to a less nutritious food product to ensure the animal obtains a nutritious and palatable meal. The dressing contains vitamin E, vitamin C and taurine.

The present invention provides, amongst others, a means to overcome the problem of oxidative stress in the domestic cat and dog.

Accordingly, a first aspect of the invention provides a dog or a cat foodstuff, diet or supplement which provides a concentration of vitamin E at a level of 25IU/400kcal or above, a concentration of vitamin C of 10mg/400kcal or above and a concentration of taurine of 80mg/400kcal or above, for use in strengthening the immune response. The amount of vitamin E is sufficient to increase the plasma vitamin E level. The increase may be to the maximum/saturation point measureable in the plasma of the animal. The increase may be in the range of 2 to 3 times the animal's own base line for plasma vitamin E levels (around the maximum physiological increase). The increase may be measured as an increase in the plasma vitamin E level of up to 25%, preferably 25% or above (preferably up to 50%, or 25 to 50%, or even 50 to 90%) of an individual animal when compared to the plasma vitamin E level when the animal is fed a control diet. The control, diet for example, is such that the total vitamin E consumption for the cat or dog is 10IU/400kcal.

Vitamin E is a collective term for several biologically similar compounds, including those called tocopherols and tocotrienols, which share the same biological activity. The most biologically active biological form of vitamin E (also the most active antioxidant) in animal tissue is alpha-tocopherol. Vitamin E cannot be synthesised *in vivo*. Vitamin E protects against the loss of cell membrane integrity, which adversely alters cellular and organelle function.

Units of vitamin E can be expressed as International Units (IU), where 1 IU of alpha-tocopherol equals 1mg of alpha-tocopherol. Other vitamin E compounds have their IU determined by their biopotency in comparison to alpha-tocopherol as described in McDowell, L.R (1989) Vitamin E: In vitamins in Animal Nutrition, Chapter 4, page 96, Academic Press, UK.

To date, levels of vitamin E above and beyond the minimum levels sufficient to prevent vitamin E deficiency symptoms present in the domestic dog or cat have not been of interest. This invention identifies that the levels of vitamin E in the dog or cat reflect the levels present in their diet and that these levels provide a typical baseline level (see examples) which is not exceeded when the animal is fed on prepared petfood. The present invention shows that the levels of vitamin E in the dog and cat can be increased by incorporating higher levels of vitamin E in the animal's diet (and that this can be achieved by the provision of specialised prepared petfood and/or a cat or dog supplement).

Aspects of the invention provide a means for reducing oxidative stress in the domestic cat and dog. Such a reduction in oxidative stress, in particular strengthens the immune response and provides a healthier animal. Markers of oxidative damage in a dog or cat include, amongst others: plasma carbonyls (end products of protein oxidation), plasma lipid hydroperoxides (markers of lipid oxidation), and anti-LDL antibodies which are produced as a response to LDL oxidation. A decline in any of these is indicative of reduced oxidative damage.

The vitamin E according to the first aspect of the invention may be in any form. It may be a tocopherol or a tocotrienol. It may be alpha-tocopherol, (d-α or dl-α) beta-tocopherol (d-β or d1-β), gamma-tocopherol (d-γ or d1-γ), delta-tocopherol, alpha-tocotrienol, beta-tocotrienol, gamma-tocotrienol or delta-tocotrienol. Preferably it is alpha-tocopherol.

The source of the vitamin E is not limiting. Preferred vitamin E sources include vitamin E acetate, (c.g tocopherol acetate), vitamin E acetate adsorbate or vitamin E acetate spray dried. Preferred sources are synthetic although natural sources may be used.

Hereinafter in this text, the term "foodstuff' covers all of foodstuff, diet and supplement. Any of these forms may be solid, semi-solid or liquid.

The supplement is particularly useful to supplement a diet or foodstuff which does not contain sufficiently high levels of one or more of the components according to the invention. The concentrations of the components in the supplement may be used to "top up" the levels in the animal's diet or foodstuff. This can be done by including a quantity of the supplement with the animal's diet or by additionally feeding the animal a quantity of the supplement. The supplement can be formed as a foodstuff with extremely high levels of one or more components of the invention which requires dilution before feeding to the animal. The supplement may be in any form, including solid (e.g. a powder), semi-solid (e.g. a food-like consistency/gel), a liquid or alternatively, it may be in the form of a tablet or capsule. The liquid can conveniently be mixed in with the food or fed directly to the animal, for example via a spoon or via a pipette-like device. The supplement may be high in one or more components of the invention or may be in the form of a combined pack of at least two parts, each part containing the required level of one or more component.

Preferably the vitamin E is incorporated into a commercial petfood product or a commercial dietary supplement. The petfood product may be a dry, semi-dry, a moist or a liquid (drink) product. Moist products include food which is sold in tins or foil containers and has a moisture content of 70 to 90%. Dry products include food which have a similar composition, but with 5 to 15% moisture and presented as biscuit-like kibbles. The diet, foodstuff or supplement is preferably packaged. In this way the consumer is able to identify, from the packaging, the ingredients in the food and identify that it is suitable for the dog or cat in question. The packaging may be metal (usually in the form of a tin or flexifoil), plastic, paper or card. The amount of moisture in any product may influence the type of packaging which can be used or is required.

The foodstuff according to the present invention encompasses any product which a dog or cat may consume in its diet. Thus, the invention covers standard food products, as well as pet food snacks (for example snack bars, biscuits and sweet products). The foodstuff is preferably a cooked product. It may incorporate meat or animal derived material (such as beef, chicken, turkey, lamb, blood plasma, marrowbone etc, or two or more thereof). The foodstuff alternatively may be meat free (preferably including a meat substitute such as soya, maize gluten or a soya product) in order to provide a protein source. The product may contain additional protein sources such as soya protein concentrate, milk proteins, gluten etc. The product may also contain a starch source such as one or more grains (e.g. wheat, corn, rice, oats, barely etc) or may be starch free. A typical dry commercial dog and cat food contains about 30% crude protein, about 10-20% fat and the remainder being carbohydrate, including dietary fibre and ash. A typical wet, or moist product contains (on a dry matter basis) about 40% fat, 50% protein and the remainder being fibre and ash. The present invention is particularly relevant for a foodstuff as herein described which is sold as a diet, foodstuff or supplement for a cat or dog.
In the present text the terms "domestic" dog and "domestic" cat mean dogs and cats, in particular *Felis domesticus* and *Canis domesticus.*

The level of plasma vitamin E in a cat or dog can easily be determined. A representative example of determining plasma vitamin E level is described in the introductory portion of the examples.

The concentration of vitamin E in a product (solid or liquid or any other form) can easily be determined. This is also described in the introductory portion of the examples.

In the first aspect of the invention, the control diet may, instead, provide a total vitamin E to the animal of 15IU/400kcal.

The concentration of the vitamin E according to the first aspect of the invention is at a level of from 25IU/400kcal diet. Throughout this text, references to concentrations per kcal arc to kcal total metabolisable energy intake. The determination of calorie density can be identified using Nutritional Requirements of Dogs (1985) National Research Council (U.S.) National Academy Press Washington DC, ISBN: 0-309-03496-5 or Nutritional Requirements of Cats (1986) National Research Council (U.S.) National Academy Press Washington DC, ISBN: 0-309-03682-8. Preferred levels for cats are from 30IU/400kcal, from 35IU/400kcal, from 40IU/400kcal, from 45IU/400 kcal, from 50TU/400 kcal, from 55IU/400kcal, up to about 100IU/400kcal or above. Preferred levels for dogs are from 30IU/400kcal, from 40IU/400kcal, from 45IU/400kcal, from 50IU/400kcal, from 55IU/400kcal, from 60IU/400kcal, from 65IU/400kcal, up to about from 100IU/400kcal or above.

For the first aspect of the invention, the foodstuff includes an amount of vitamin C (ascorbic acid).

Vitamin C is a water-soluble substance. It is synthesised *de novo* in both the domestic cat and the domestic dog. Because it is synthesised *in vivo,* the effect of vitamin C supplements in dog and cat has not previously been investigated. In particular, the effect of vitamin C supplementation in cat and dog, as a potential antioxidant and in combination with vitamin E supplementation has not been investigated.

The present invention shows that vitamin C levels in a cat or a dog can be increased by supplementation. This is demonstrated by an increase in plasma values following vitamin C supplementation. The increase in vitamin C levels can contribute to a reduction in free radicals and therefore a reduction in oxidative stress in the animal.

The vitamin C according to the first aspect of the invention may be in any form. It may be liquid, semi-solid or solid. Preferably it is a heat stable form such as a form of calcium phosphate.

The source of the vitamin C is not limiting. Preferred vitamin C sources include crystalline ascorbic acid (optionally pure), ethylcellulose coated ascorbic acid, calcium phosphate salts of ascorbic acid, ascorbic acid-2-monophosphate salt or ascorbyl-2-monophosphate with small traces of the disphosphate salt and traces of the triphophate salt, calcium phosphate, or for example, fresh liver.

The level of vitamin C in a product (solid, liquid or any other form) can easily be determined. This is described in the introductory part of the examples.

A further useful point in relation to the use of vitamin E in combination with vitamin C is their potential to act synergistically. This may be assisted by the fact that vitamin E is lipid soluble and vitamin C is water-soluble. Alpha-tocopherol is known to sit in the lipid membrane. Ascorbate and alpha-tocopherol, for example, interact at the interface between cell membranes or lipoproteins and water. Ascorbic acid rapidly reduces alpha-tocopherol radicals in membranes to regenerate alpha-tocopherol. The preferred concentration of vitamin C according to the first aspect of the invention is a level which preferably increases the plasma vitamin C level of an animal by up to about 25% (preferably 25% or more) in comparison with when the animal is fed a control diet, such that its total vitamin C consumption is (for both a cat or a dog) 5mg/400kcal diet. Levels of vitamin C which do not achieve this increase are still covered by the first aspect of the invention. Levels of vitamin C according to the first aspect of the invention include from 10, 12, 15, 17, 20, 22, 25, 27, 30, 32, 38, 40, 42, 48 up to about 50 mg/400kcal diet. Preferred levels for the cat are the above options from 10 to 48 mg/400kcal and for the dog, the above options from 12 to 50 mg/400kcal. Levels above 55 mg/400kcal provide no added benefit and arc usually best avoided.

The first aspect of the invention includes an amount of taurine. The taurine is in addition to the supplemented vitamin C described above.

Taurine is an unusual amino acid found in a wide variety of animal species. Taurine is an essential nutrient for the cat which, unlike the dog, is unable to synthesise taurine from precursor amino acids. It is thought that taurine protects cellular membranes from toxic components including oxidants. The increase in vitamin taurine levels in an animal diet can contribute to a reduction in free radicals and therefore a reduction in oxidative stress in the animal, in particular in combination with the other components of the invention.

The taurine according to the first aspect of the invention may be in any form. It may be powered, crystalline, semi-solid or liquid.

The source of the taurine is not limiting. Preferred taurine sources include aminoethylsulfonic acid (C2H7N03S). Sources may be natural or synthetic.

Suitable concentrations of taurine for use according to the first aspect of the invention are usually determined, to some extent as to the processing of the product (for example, whether the product is dry or canned). To maintain plasma taurine levels in the cat at the normal range (>60µmol/l), a canned (moist) diet must supply at least 39mg of taurine/kg body weight per day and a dry diet at least 19mg/kg body weight per day. The first aspect of an invention provides, for a product which is not subjected to a high temperature method (such as canning) a preferred level of from about 80mg/400kcal, more preferably from about 100, increasing even more preferably from 120, 150, 180, 200, 220, 250, 280, 300, 320, 350, 400 and above in mg/400kcal diet. In a product which is processed such as by high temperature, levels according to the invention are preferably from about 380mg/400kcal, more preferably from about 400, increasing even more preferably from 420, 450,480, 500, 520, 550, 580, 600, 620, 650, 700 and above in mg/400kcal diet.

The concentration of taurine in a product (solid liquid or in any other form) can be easily determined. A representative method is described in the introductory portion of the examples. The *in vivo* feline status of taurine can be enhanced through dietary supplementation. The dose response effect of dietary taurine content can be measured by plasma levels. This is also described in the introductory portion of the examples.

The first aspect of the invention may further include an amount of a carotenoid. The carotenoid may be in addition to the supplemented vitamin C and the taurine as described above.

The carotenoids are a group of red, orange and yellow pigments predominantly found in plant foods, particularly fruit and vegetables, and in the tissues of animals which eat the plants. They are lipophilic compounds. Some carotenoids act as a precursors of vitamin A, some cannot. This property is unrelated to their antioxidant activity. Carotenoids can act as powerful antioxidants. Carotenoids are absorbed in varying degrees by different animal species. Carotenoids may be classified into two main groups; those based on carotenes and those based on xanthophylls (which include oxygenated compounds). Common carotenoids include; beta-carotene, alpha-carotene, lycopene, lutein, zeaxanthin and astaxanthin. Carotenoids are not proven to be essential nutrients in the feline or canine diet. Unlike humans and dogs, the cat is unable to convert the precursor beta-carotene into the active vitamin A form since the required enzyme necessary for this conversion is absent from the intestinal mucosa in cats (they do not possess the dioxygenase enzyme which is needed to cleave the carotene molecule).

This invention shows that carotenoids can be absorbed by the domestic cat and dog (to give an increased plasma concentration) and can contribute to a reduction in oxidative stress. Further, the present invention has demonstrated that the carotenoids can be absorbed following their incorporation into a commercial product. As mentioned above, the components of the first aspect of the invention may act synergistically. Vitamin E is able to protect beta-carotene from oxidation and may have a sparing effect on beta-carotene. Vitamin E is thought to protect the chemical bonds of beta-carotene from being oxidised.

The source of the carotenoids is not limiting and can include natural and synthetic sources. In particular, the preferred source is a natural source and includes; marigold meal and lucerne meal (sources of lutein); tomato meal, red palm oil, tomato powder, tomato pomace/pulp (sources of beta-carotene and lycopene). Sources include oils high in carotenoid levels and pure manufactured carotenoids such as lutein, violaxanthin, cryptoxanthin, bixin, zeaxanthin, apo-EE (Apo-8-carotenic acid ethylester), canthaxanthin, citranaxanthin, achinenone, lycopene and capsanthin. Preferred levels of total carotenoids are from 0.01mg/400kcal, or from 0.2mg/400kcal or from 1mg/400kcal or from 2mg/400kcal.

The concentrations of the following carotenoids are preferably:
Beta-carotene: 0.01 to 1.5mg/400kcal, preferably 0.5 to 1mg/400kcal
Lycopene: 0.01 to 1.5mg/400kcal, preferably 0.5 to 1mg/400kcal
Lutein: 0.05 to 1.5mg/400kcal, preferably 0.5 to 1mg/400kcal.
In particular, the present invention provides for a combination of carotenoids in the first aspect of the invention.

Preferred sources of the combined carotenoids include;
Red Palm Oil and Marigold Meal
Tomato Powder, Marigold Meal and Lucerne
Tomato Pomace and Marigold Meal.

As described above, the invention includes vitamin E and optionally other components. Useful combinations of the components (preferably in a canned or dry petfood) include;
Vitamin E, vitamin C, taurine, red palm oil and marigold meal
Vitamin E, vitamin C, taurine, tomato powder, marigold meal and lucerne
Vitamin E, vitamin C, taurine, tomato powder and marigold meal
Vitamin E, vitamin C, taurine, tomato powder and lucerne
Vitamin E, taurine, tomato pomace and marigold meal.

A combination of the present invention is;

| | Approx. active component mg/400kcal after production (Dry Product) |
|---|---|
| Vitamin C | 20mg ascorbic acid |
| Vitamin E | 50 IU |
| Taurine | 200mg (500 mg in wet product) |
| Lutein | 0.17mg |
| Lycopene | 0.03mg |
| Beta-carotene | 0.01 mg |

A further useful combination of the present invention is:

| | |
|---|---|
| Vitamin E | 50IU/400kcal |
| Vitamin C | 20mg/400kcal |
| Taurine | 500mg/400kcal |
| Beta-carotene | 0.5 to 1mg/400kcal |
| Lycopene | 1mg/400kcal |
| Lutein | 0.5 to 1mg/400kcal |

Other useful components of the foodstuff according to the invention, include; trace minerals (not direct antioxidants, but function as cofactors within antioxidant metalloenzyme systems), selenium (an essential part of the antioxidant selenoenzyme, glutathione peroxidase), copper, zinc and manganese (forming an integral part of the antioxidant metalloenzymes Cu-Zn-superoxide dismutase and Mn-superoxide dismutase.

In accordance with the second aspect of the invention, the components may be consumed simultaneously, separately, or sequentially.

Preferably, the dog or cat foodstuff provides an antioxidant status of greater than 20mg/l of vitamin E.

The invention provides a dog or cat foodstuff which provides a concentration of vitamin E at a level according to the first aspect of the invention. The concentration may be as stated according to the first aspect of the invention which provides the described percentage increases or the particular (including preferred) levels.

The dog or cat foodstuff also provide a concentration of vitamin C at a concentration also according to the vitamin C levels of the first aspect of the invention.

The dog or cat foodstuff provides, in addition to the vitamin C, a concentration of taurine at a concentration also according to the taurine levels of the first aspect of the invention.

The dog or cat foodstuff may provide, in addition to the vitamin C and the taurine a concentration of a carotenoid at a concentration also according to the carotenoid levels of the first aspect of the invention.

This aspect of the invention further provides a dog or cat foodstuff for use in the prevention or treatment of any disorder which has a component of oxidative stress. The use is separately for the prevention or treatment of oxidative stress as a component of a "disease" or "disorder" (thus the disease or disorder may be reduced by alleviating (at least to an extent) a component of oxidative stress), Such disorders include; ageing, cancer, heart disease, atherosclerosis, arthritis, cataracts, inflammatory bowel disease, renal disease, renal failure, neurodegenerative disease and immunity (such as compromised immunity). Also included are prevention and treatment of oxidative stress caused by animal vaccinations (often annually) and anaesthetics, which may also be used for annual procedures such as dental treatments (which may require general anaesthetic) and exposure to UV light or radiation. With respect to immune function, this is equally applicable to those subjects who have a compromised immune function due to age (e.g. growing animals or senescing animals) as well as those experiencing immunological challenge. The maintenance of a healthy immune response (as well as optimising or boosting an immune response) in animals who are clinically healthy is also included in this definition.

The immune system of vertebrate animals is much discussed in the art (for example "Immunology" by Roitt, Brostaff and Male, Gower Medical Publishing, London, New York, 1985). Immunological challenge includes infection, vaccination and other external factors such as anaesthesis (for example prior to surgery). It is an object of the present invention to provide a diet/foodstuff or supplement (and related aspects) which can be used to maintain, optimise or "boost" the immune system such that an improved immune response is given on an immunological challenge. An immune response can be monitored by measuring antibodies produced in response to a given antigen. Such knowledge and technology is standard in the art. An improved immune response may be represented by a higher level (titre) of circulating antibodies within a given time frame, a faster detected antibody response or maintenance of the circulating antibody titre for a longer period of time.

An improved immune response assists the animal during an immunological challenge and can be particularly useful for young animals, since young animals may not have a fully developed immune system. As young animals are often vaccinated, the present invention provides means by which an improved immune response can be given by the animal when vaccinated. The present invention is particularly useful for feeding to a dog prior to vaccination with vaccine antigens for distemper, parvovirus and/or adenovirus. The present invention is particularly useful for feeding to a cat for vaccines or a combined vaccine against Feline Panleucopenia, Feline Calicivirus and/or Feline Herpesvirus. The length of time suitable for feeding prior to immune challenge depends on the animal in question and the immunological challenge. The foodstuff can be fed consistently. Periods of 8, 6, 4, 2 and 1 week prior to immune challenge are suitable. Longer periods are also suitable.

A further aspect provides for the use of vitamin E, vitamin C and taurine in the manufacture of a foodstuff, diet for or supplement strengthening the immune response of a cat or a dog.

Preferred features of aspect one also applies to this aspect.

A preferred feature of this aspect of the invention provides for the use of vitamin E at a level of 25IU/400kcal or above, incorporated into a foodstuff as an *in vivo* antioxidant, in a dog or a cat. In particular, the levels of vitamin E may be as the preferred levels for vitamin E set out for the first aspect of the invention.

A third of the invention provides a method for making a foodstuff according to any of the first and second aspects of the invention the method comprising mixing together at least two ingredients of the foodstuff. Some of the components will be the required level of vitamin E vitamin C and taurine. The preferred form of the foodstuff is a petfood product and therefore the method for making the petfood product, in any form, comprises mixing together the ingredients for the petfood product and incorporating one or more of the components according to the invention. The components may be added at any time during the manufacture/processing of the foodstuff, including at the end, as the last step before packaging.

The product can be made according to any method known in the art, such as in Waltham Book of Dog and Cat Nutrition, Ed. ATB Edney, Chapter by A. Rainbird, entitled "A Balanced Diet", pages 57 to 74, Pergamon Press.

This aspect of the invention provides a dog or cat foodstuff also comprising vitamin C at a concentration of from 15mg/400kcal diet. The diet, foodstuff or supplement details are as those described for the previous aspects of the invention in relation to the vitamin C component to the extent that it comprises a vitamin C concentration of from 15 mg/400kcal diet. Features of aspects one and two, as herein described may be individual or combined options together with the vitamin C concentration according to the third aspect of the invention. The second aspect of the invention provides a foodstuff useful for the prevention or treatment of a disorder which has a component of oxidative stress. Such disorders are also those as described above for the previous aspects of the invention. The inclusion of vitamin C in a dog or cat foodstuff is unique in as far as it relates to the concentrations of vitamin C stated and in particular or for the uses given.

The vitamin C concentrations range from 15mg/400kcal upwards. Preferred levels are those above 15mg/400kcal as set out above according to the preferred concentrations of vitamin C according to the first aspect of the invention. Because vitamin C is synthesised *in vivo* in both the domestic cat and the domestic dog it has never been of particular interest to consider introducing to a cat or dog supplemental levels of vitamin C via cat or dog food. However, the present invention shows that such a diet can be particularly useful, primarily for the production of a clinical diet/veterinary diet/medicament.

The present invention also provides for the use of vitamin C in a foodstuff for a dog or a cat. The use may be in the manufacture of a diet for the prevention or treatment of a disorder which has a component of oxidative stress or for the prevention or treatment of the oxidative stress component. Those disorders include cancer, ageing, heart disease, atherosclerosis arthritis, cataracts inflammatory bowl disease, renal disease, renal failure, neurodegenerative disease or compromised immunity, for example, an animal suffering from an infection. The present invention may also be used to treat or assist in the event of an immunological challenge in healthy animals.

The following figures are referred to in the examples section:
Fig. 1, which shows an increase in vitamin E plasma status in dogs through supplementation with 50IU/400kcal and 100IU/400kcal of vitamin E.
Fig. 2, which shows vitamin C plasma status in cats, reflecting dietary vitamin C supplementation.
Fig. 3, which shows vitamin E plasma status in cats with dietary vitamin E supplementation.
Figs. 4 and 5, which show levels of anti-parvovirus antibody titres with supplemented and unsupplemented diets, post vaccination
Fig. 6, which shows an anti-distempter vaccine response with supplemented and unsupplemented diets, post vaccination.
Fig. 7, which shows maintenance of anti-adenovirus antibody titres in dogs supplemented with an antioxidant cocktail.
Fig. 8, which shows the measurement of FRAP in dogs fed an antioxidant diet for 8 weeks.
Fig. 9, which shows plasma vitamin E levels in dogs fed an antioxidant diet for 8 weeks.

The invention will now be described with reference to the following examples.

### EXAMPLES

### Introductory Portion

This section describes, firstly, how blood samples may be taken for determination of vitamin E, vitamin C, taurine and carotenoids. Also described are methods for analysis of components in plasma and methods for measuring components in food. In addition to the details set out below, details regarding analytical procedures can be found in McDowell L.R. (1989) Vitamin E: In Vitamins in Animal Nutrition Chapter 4, page 96, Academic Press, UK.

### Plasma and Whole Blood Taurine

### Preparation of samples:

Blood samples are collected into heparinarised bottles from either the cephalic or jugular vein. Following mixing of the sample on a roller, the samples are kept on ice for transfer to the laboratory. Whole blood is then frozen at -20°C until analysis. Alternatively for plasma measurement, plasma is extracted by centrifugation of blood samples (at 3500 rpm for 10 minutes at 0°C). Plasma is frozen at -20°C until analysis.

The analysis of Taurine in cat plasma/blood is carried out by taking the sample and precipitating out protein by reaction with sulpho-salicylic acid solution. The sample is then centrifuged and the supernatant liquor filtered.

### Reference where plasma taurine has been measured in cats:

Earle, K.E. and Smith, P.M. (1991) The effect of dietary taurine content on the plasma taurine concentration of the cat. British Journal of Nutrition 66, 227-235.

### Plasma Vitamin C

### Preparation of samples:

Blood samples are collected into heparinarised light-protected (foil-wrapped) bottles from either the cephalic or jugular vein. Following mixing of the sample on a roller, the samples are kept on ice for transfer to the laboratory. Plasma is extracted by centrifugation of blood samples (at 3500 rpm for 10 minutes at O°C). Plasma is frozen at -20°C until next-day analysis. Samples are prepared under subdued lighting at all times.

1ml plasma extracted with 5ml extractant (15g metaphosphoric acid + 0.475g EDTA + 20ml glacial acetic acid in 500ml water) - the procedure is then the same as for product.

A preferred minimal dose of vitamin C to achieve an increase in plasma in cats is 20mg/400kcal. A preferred minimal dose of vitamin C tested to achieve an increase in plasma in dogs was 27mg/400kcal.

### Plasma Vitamin E

### Preparation of samples:

Blood samples are collected into heparinarised bottles from either the cephalic or jugular vein. Following mixing of the samples on a roller, the samples are kept on ice for transfer to the laboratory. Plasma is extracted by centrifugation of blood samples (at 3500 rpm for 10 minutes at 0°C). Plasma is frozen at -20°C until analysis.

Sample size = 250µl. The sample is extracted into hexane after the addition of tocopherol acetate as internal standard. The hexane is evaporated and the residue dissolved in methanol and injected onto the HPLC. Separation is achieved using a reverse-phase column with methanol as eluent with UV detection at 285nm.

A preferred minimal dose of vitamin E to achieve an increase in plasma in cats is 34 IU/400kcal. A preferred minimal dose of vitamin E tested to achieve an increase in plasma in dogs was 50IU/400kcal.

### Carotenoid determination in plasma

Blood samples are collected into heparinarised light-protected (foil-wrapped) bottles from either the cephalic or jugular vein. Following mixing of the samples on a roller, the samples are kept on ice for transfer to the laboratory. Plasma is extracted by centrifugation of blood samples (at 3500 rpm for 10 minutes at 0°C). Plasma is frozen at -80°C until analysis. Samples are prepared under subdued lighting at all times.

The following two methods may be used to determine carotenoid concentration in plasma.

### Method 1

The first method is to measure the major carotenoids of interest, with the exception of lutein and zeaxanthin which will not be separated using this method.

The method used to detect carotenoids is a variation of that of Craft, N.E. and Wise, S.A., Journal of Chromatography, 589, 171-176, (1992).

The extraction of carotenoids from plasma is achieved using a variation of that of Thurnham *et. al.* Clinical Chemistry, 34, 377-381, 1988.

### Method 2

The second method is to separate lutein and zeaxanthin and to separate the different isoforms of the carotenoids.

The method used to detect the carotenoids is a variation of that of Yeum, Kyung-Jin., *et. al.* Am. J. Clin. Nutr, 64, 594-602, 1996.

The extraction of carotenoids from plasma is achieved using a variation of that of Thurnham *et. al.* Clinical Chemistry, 34, 377-381, 1988.

All extractions were carried out under subdued lighting, and all stock solutions of carotenoids were stored under argon.

### Vitamin C - Food Product

Ascorbic acid is enzymatically oxidised to dehydro ascorbic acid which is condensed with o-phenylene diamine to the fluorescent quinoxaline derivative. The latter is separated from interfering compounds by reversed-phase HPLC with fluorimetric detection.

### Vitamins A & E Food Product

The sample is hydrolysed with ethanolic potassium hydroxide solution and the vitamins extracted into petroleum ether. The petroleum ether is removed by evaporation and the residue is dissolved in propan-2-ol. The concentration of vitamin A and E in the propan-2-ol extract is determined by reversed-phase liquid chromatography.

### Free Taurine - Food Product

Free Taurine is that which is nutritionally available in a product.

The analysis of Free Taurine is carried out by taking the sample, adding dilute Hydrochloric acid. This is then macerated and transferred to a volumetric flask. A small amount is then taken and sulpho-salicylic acid is added to precipitate the protein. The sample is then centrifuged and the supernatant liquor filtered. The resulting solution is reacted with dansyl chloride and analysed by HPLC using fluorescence detection.

### Carotenoids - Food Product

20-25g sample taken for analysis. Sample is saponified with 28% ethanolic potassium hydroxide for 30mins. At 90°C under nitrogen and with pyrogallol as antioxidant. After cooling, the saponified extract is extracted with 2x250ml mixed ethers (pet. Ether/diethyl ether 1:1) and the organic phase is washed with water until neutral. The ether extract is evaporated at 35°C under vacuum with BHT as antioxidant and the residue redissolved in the HPLC mobile phase. The carotenoids are determined using reverse phase HPLC using UV detection at 450nm.

In addition to the experimental work given, the invention was an indicator of improved health by decreasing the osmotic fragility of cat erythrocytes following feeding of the antioxidant cocktail to cats.

The ability of red blood cells (erythocytes) to withstand osmotic stress was tested. The method involved re-suspension of washed erythrocytes in solutions with different NaCl concentrations; these are incubated and then centrifuged. Haemoglobin is released from the cells according to their osmotic fragility. Results showed that erythocytes of cats fed antioxidant cocktails of the invention showed a greater resistance against osmotic stress as significant lower concentrations of NaCl were required to induce the same level of heamolysis. The ability of erythocytes to tolerate situations of osmotic stress is an indicator of an improved physiological status.

### Comparative Example 1

### Vitamin E

A group of 8 dogs were maintained a nutritionally complete diet (see reference section representative diet) for a period of 6 weeks prior to receiving one of the two supplementary levels of vitamin E (alpha-tocopherol acetate below (table 1)).

**Table 1: Vitamin E content in test diet before supplementation**

| Diet | Vitamin E content before Supplementation |
|---|---|
| Complete and balanced | 8.7 IU/400kcal |

Plasma levels significantly (p<0.05) increased following only 1 week of supplementation at both the 50IU/400kcal and 100IU/400 kcal level.
Plasma saturation appeared to occur after 1 week of supplementation. Plasma levels declined to baseline levels after 2 weeks of stopping supplementation.

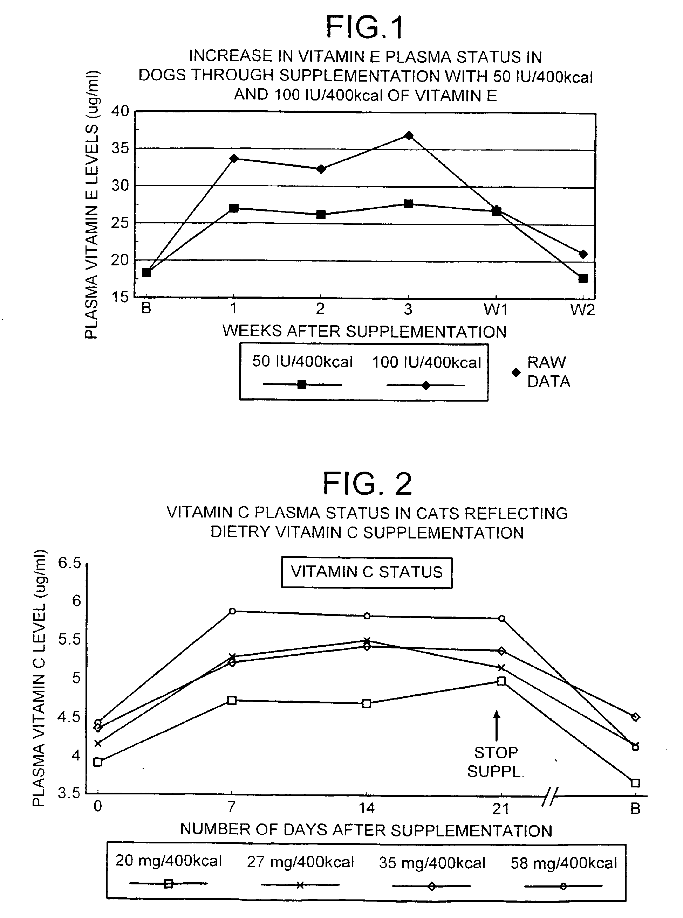

It can be concluded from the doses studied, that dietary supplementation with vitamin E significantly increases plasma status in dogs by a magnitude of 60-66% (figure 1).

### Comparative Example 2

### Antioxidant Supplementation in Cats

### Vitamin C

4 groups of 8 or 9 cats were given oral supplements of vitamin C at 4 different levels whilst being maintained on a nutritionally complete diet (see reference diet section).

Baseline plasma vitamins C levels demonstrate that there are no significant differences between the groups prior to supplementation.

As can be seen in figure 2, within 7 days the plasma vitamin C levels for all 4 groups significantly increased above their baseline prior to supplementation.

Although the higher vitamin C supplements resulted in greater plasma values, the increase was not significantly different between the 4 groups.

Following the end of the supplementation period plasma vitamin C levels returned to baseline.

These data demonstrate that the antioxidant status of cats can be increased with vitamin C supplementation of their diet at relatively low levels.

### Comparative Example 3

### Vitamin E

The impact of typical dietary vitamin E levels on the vitamin E status of cats has been evaluated, as well as the effect of dietary supplementation.

The plasma vitamin E concentrations of 2 groups of 12 cats maintained on 2 commercial nutritionally complete cats diets (see reference diet section) with different dietary vitamin E levels were determined. The plasma levels (duplicate measurements 2 weeks apart) seen in Table 2 demonstrate that cats maintained on a diet for a period of 4 weeks with a higher vitamin E level have a relatively increased vitamin E status.

**Table 2: Vitamin E plasma status in cats reflecting vitamin E intake**

| Vitamin E content | Plasma Vitamin E (±SD) ug/ml |
|---|---|
| 60IU/400kcal | 26.62±7.2 |
| 24IU/400kcal | 15.09±4.0 |

In order to determine the effect of supplementation, oral vitamin E supplements (α-tocopherol acetate) were administered to a group of 12 cats whilst being maintained on the nutritionally complete diet.

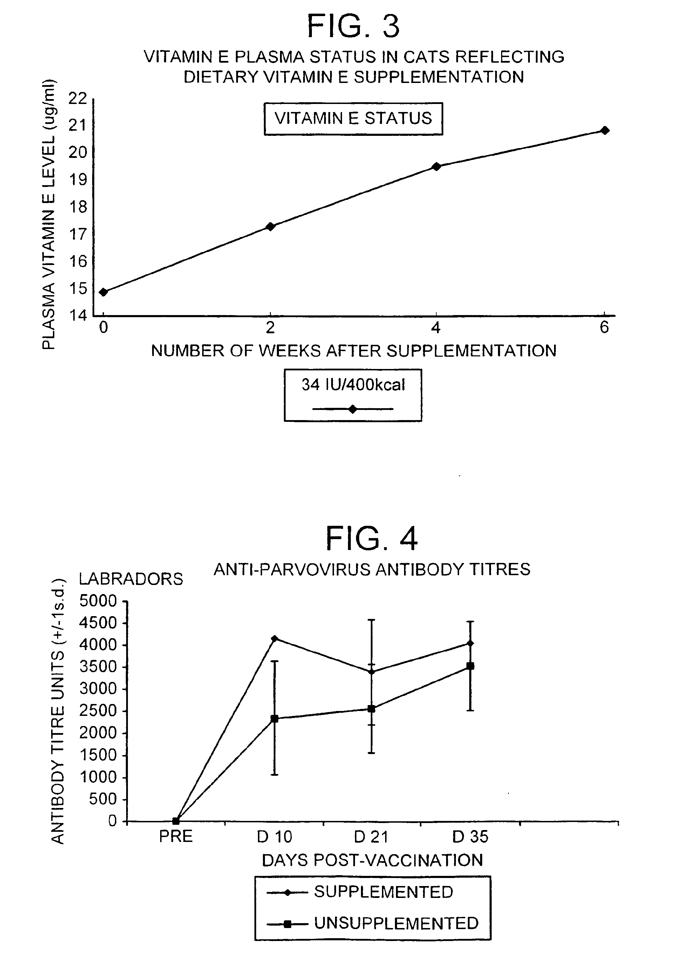

The effect of the supplementation can be seen in figure 3. Plasma vitamin E levels were significantly increased following supplementation, reaching saturation after approximately 4 to 6 weeks of supplementation.

Hence, dietary vitamin E supplementation can enhance the antioxidant status of cats.

### Comparative Example 4

Total plasma antioxidants in cats; normal ranges and influence of age.

The total antioxidant status in the domestic cat has never previously been reported. The aim of this study was to validate a method of determining total antioxidant status in the cat, determine normal total antioxidant ranges in healthy adult cats and investigate the effect of age on total plasma antioxidant status.

A colorimetric assay kit (NX2332) manufactured by Randox Laboratories Limited, was validated for the determination of total antioxidant status in the cat. Assay precision was determined both by replicate analysis of cat plasma samples, and of commercial quality control (QC) material (PAR 721013 and PAR 721014; Bio-stat Diagnostics), Intra and Inter assay coefficient of variation (CV) demonstrated acceptable variation for spectrophotometric assays with plasma sample CV < 10%, and QC CV < 5%. Dilution of cat plasma produced a linear response in the assay.

The effect of ageing on plasma antioxidant levels was evaluated. 134 cats, maintained on a range of nutritionally complete cat foods (see diet reference section), participated in the study. Total antioxidant status was evaluated in single plasma samples from 69 male and 65 female domestic shorthaired cats aged between 6 months and 14 years. All means are quoted ± the standard deviation (SD).

The results indicate the presence of a transitional period of antioxidant status occurring at approximately 6 years of age. A Newman-Keuls multiple range test showed that the total plasma antioxidant status of cats aged under 6 years was significantly (p<0.01) higher than those aged over 6.5 years (see Table 3). It is not known whether a decline in antioxidant status at this age signals a greater susceptibility to illness or whether antioxidant status in this age influences lifespan in cats.

**Table 3. Mean total plasma antioxidant concentrations from cats categorised according to age.**

| Age Category (years) | Total Plasma Antioxidant Status (mmol/1) ± SD | Number of animals |
|---|---|---|
| <6 | 0.920±0.77^{a} | 47 |
| 6 | 0.872±0.182^{ab} | 9 |
| >6 | 0.799±0.092^{b} | 78 |

| | | |
|---|---|---|
| *Superscript denote homogeneous groups* | | |

### Comparative Example 5

### Total plasma antioxidant and superoxide dismutase status in dogs

Little is known about the antioxidant status in the domestic dog. The aim of this study was to validate a method of, and determine normal ranges of, total plasma antioxidants (TPAO) and erythrocyte SOD (superoxide Dismutase) activity in the dog.

Colorimetric assay kits manufactured by Randox laboratories Limited were validated for determination of TPAO status (Kit No: NX2332 and SOD status (Kit No: SD 125 in the dog. For TPAO, assay precision was determined both by replicate analysis of dog plasma samples, and, of commercial quality control (QC) material (PAR 721013 and PAR 721014: biostat Diagnostics). Inter and Intra assay coefficient of variation (CV) demonstrated acceptable variation for spectrophotometric assays, with plasma sample CV<10% and QC<5%. SOD status assay precision was determined by replicate analysis of dog erythrocyte samples. Inter and Intra assay coefficient of variation (CV) demonstrated acceptable variation for spectrophotometric assays with erythrocyte sample CV<10%.

TPAO and erythrocyte SOD status were evaluated in 30 male and 20 female dogs aged between 9 months and 16 years. Ten breeds were represented. All dogs were clinically normal, and had been maintained long-term on a variety of manufactured nutritionally complete diets. There was no apparent relationship between age and TPAO or SOD activity. Dogs were not equally represented across the age groups (data not shown), and further work is in progress to assess whether this observation is supported by a larger sample size and better age spread. No significant difference was seen between the gender for either mean TPAO status (male: 0.719±0.135, female: 0.786±0.101) or SOD activity (male: 1275.41 ±264.46, female: 1267.61±166.34 U/g Hb). However, Bartletts Test showed a significant difference (p<0.05) between variance of SOD activity in male (6994.0) and female (27670.3) dogs.

Analysis of those breeds with five or more representatives showed a significant difference (p<0.01) for both SOD activity, and TPAO status, between breed but not gender. Newman-Keuls multiple range tests showed Beagles to have significantly lower mean SOD activity (p<0.05) and mean TPAO status p<0.01) than Labradors and Yorkshire Terriers, Table 4.

**Table 4. Superoxide dimutase and total plasma antioxidant activity for three breeds of dog.**

| Breed | Mean SOD activity ± SD | Mean TPAO activity ± SD | n |
|---|---|---|---|
| Beagle | 1084.70±136.24^{a} | 0.569±0.094^{a} | 10 |
| Labrador | 1323.65±185.77^{b} | 0.830±0.113^{b} | 15 |
| Yorkshire Terrier | 1293.76±215.54^{b} | 0.798±0.036^{b} | 5 |

| | | | |
|---|---|---|---|
| *Superscripts denote homogeneous groups within columns (Newman-Keuls multiple range test)* | | | |
| *n*=*number of animals* | | | |

These data indicate that it may not be sufficient to assume a single value for normal SOD and TPAO ranges in dogs. In this instance two values are required, one for Beagles and one for the other two breeds. The 95% confidence intervals for the mean ranges of TPAO status and erythocyte SOD activity in Beagles are 0.569 ± 0.067 mmol/l and 1084.9 ± 97.27 U/g Hb respectively, and for the other two breeds; 0.822 ± 0.047 mmol/l and 1316.2 ± 88.03 U/g Hb.

### Comparative Example 6

### Assessment of total antioxidant status in the cat and dog using a fully automated colormetric assay

The aim of this study was to validate a colormetric assay kit, manufactured by Randox Laboratories Ltd, for the determination of total antioxidant status in the cat and dog. Secondly the validated assay was used to assess changes in total antioxidant status of the cat and dog with age and to compare these changes in other biochemical and haematological parameters. The assay reacts 2,2'-Azino-di-(3-ethylbenzthiazoline sulphonmate) (ABTS) with a peroxide and H₂O₂ to produce a radical cation ABTS⁺ which can be measured to 600nm. Antioxidants present in a sample will cause suppression of this colour production to a degree proportional to their concentration. The assay was performed on a Cobas Mira analyser (Roche Diagnostics) using the protocol supplied by Randox Laboratories. Assay precision was determined by replicate analysis of cat and dog plasma samples and of commercial quality control (QC) material. Inter-assay and intra-assay CV were both less than 5% for the QC material and were both less than 10% for the cat and dog plasma samples. Dilutions of cat and dog plasma produced a linear response in the assay. Routine haematology and biochemistry profiles, including total antioxidant status, were performed on domestic short haired cat and on several breeds of pedigree dogs of various ages. Antioxidant status in the cat increased up to 2 months of age, but then showed a subsequent decline. No sex difference were apparent. Female dogs of less than 1 year showed a slight decrease in antioxidant status, otherwise there was no significant change with age. Multiple regression analysis demonstrated a relationship between antioxidant status, albumin, asparate aminotransferase and calcium in the cat and antioxidant status, phosphate and alanime aminotransferase in the dog. The validation results were considered to be acceptable and the assay is suitable for the determination of total antioxidant status in the cat or dog.

### Comparative Example 7

Effect of a vitamin C supplement on plasma status in healthy adult cats.

Vitamin C is a major water soluble antioxidant *in vivo,* that can delay or inhibit oxidation, important particularly in extracellular fluids. However the response in the cat *(Felis domesticus)* to different dietary levels of vitamin C has not been previously investigated. The aim of this study was to establish the effect of vitamin C supplementation in healthy adult cats on plasma status.

Plasma vitamin C concentrations of 33 cats were determined by reversed-phase High Performance Liquid Chromatography. Subsequently the cats were allocated into 4 groups by stratified randomed sampling, ensuring there were no significant differences between the baseline plasma vitamin C levels of the 4 groups (ANOVA p>0.05). Daily vitamin C supplements (crystalline L-ascorbic acid, ICN Pharmaceuticals, UK, in a gelatine capsule, Analytical Supplies Limited, UK) were given orally to the cats for 21 days. The 4 groups of cats received either 3.5, 7.0, 10.5 or 21.0 mg vitamin C/day. Plasma vitamin C levels were determined at 7, 14 and 21 days of supplementation, as well as, at 21 days post-supplementation. All cats were maintained on a nutritionally complete canned diet, with a vitamin C content of 11.6 mg/1.6MJ.

The plasma vitamin C levels of the cats significantly increased at 7 days after daily supplementation at all 4 dietary levels administered (paired t-test p<0.05), and during the supplementation period these levels were maintained, Table 5. Although the higher dietary supplementation levels of vitamin C achieved greater plasma values when compared with those of the lower dietary levels, there were no significant differences between dietary groups in the plasma increases (repeated measures multifactor ANOVA using General Linear Model p<0.05). The plasma levels of all cats returned to baseline levels 21 days post-supplementation.

**Table 5. Plasma Vitamin C Concentrations (ug/ml ± SD)**

| Group | n | Time of supplementation (days) | | | | 21 days post-supplementation |
|---|---|---|---|---|---|---|
| | | 0 | 7 | 14 | 21 | |
| 1 | 8 | 3.93^{a}±0.98 | 4.73^{b} 0.71 | 4.73^{b}±0.39 | 5.02^{b}±0.64 | 3.66^{a}±0.74 |
| 2 | 8 | 4.16^{a}±1.18 | 5.28^{a}±0.99 | 5.55^{b}±0.67 | 5.20^{b}±0.82 | 4.17^{a}±0.73 |
| 3 | 8 | 4.34^{a}±1.24 | 5.20^{a}±1.16 | 5.46^{b}±1.02 | 5.42^{b}±1.28 | 4.56^{a}±1.14 |
| 4 | 9 | 4.41^{a}±0.96 | 5.91^{b}±1.04 | 5.90^{b}±0.54 | 5.85^{b}±0.80 | 4.17^{a}±0.65 |

Heterogeneous superscripts within rows indicate a significant difference with time (ANOVA p<0.05)

These data demonstrate that at low levels of dietary supplementation with vitamin C administered on a daily basis, the plasma status of cats can be significantly enhanced. Continual supplementation is required in order to maintain the enhanced plasma status.

### Comparative Example 8

Validation and normal ranges of plasma ceruloplasmin concentration in cats and dogs.

The cuproenzyme, ceruloplasmin has a number of functional roles with the body. As a copper storage protein, ceruloplasmin helps protect against the catalytic ability of free copper ions. Free copper can accelerate autoxidation reactions through single-electron (radical) transfer, as well as react with hydrogen peroxide to form highly reactive hydroxyl radicals which can lead to cellular disruption. At time of trauma, ceruloplasmin also promotes the conversion of iron from its pro-oxidant ferrous form to ferric iron. As an acute phase protein, ceruloplasmin can be indicative of inflammation or infection and thus be used in conjunction with serum ferritin as a measure of iron stores.

A colorimetric method to determine ceruloplasmin oxidase activity in cats and dogs was validated based on the method of Sunderman F.W. and Nomoto, S. (1990) Clin. Chem 16, 903 using p-phenylenediamine as the substrate. Two dog and two cat serum samples were analysed ten times within a single run. Intra-assay coefficient of variation of 1.94% and 2.95% were determined for the dog samples, and 1.81 % and 3.94% for the cat. Analysing the same samples on ten separate days, inter-assay coefficients of variation of 8.21% and 7.01% for dog, 6.88% and 9.35% for cat samples were determined. Hence an acceptable level of intra- and inter-assay variability was achieved. Following this, the difference between the ceruloplasmin concentration of serum and plasma samples was evaluated. No significant difference was determined between plasma and serum samples for either species. Hence thereafter plasma samples were collected in order to reduce the total volume of sample required.

In order to establish normal ranges, plasma samples were obtained from 102 healthy dogs (mixed breed, age and sex) and 54 healthy domestic short-haired cats (mixed age and sex). The mean plasma ceruloplasmin concentrations determined for dog and cat where 9.28 IU/L (SD 3.03 IU/L) and 10.90 IU/L (SD 3.34 IU/L) respectively. Using these values, normal ranges (mean ±2SD) of 3.22 IU/L 15.35 IU/L for dogs, and 4.22 IU/L to 17.58IU/L for cats were established. This normal range determined for dogs is consistent with that previously reported (Solter P.F, et al (1991) Am J Vet Res 52, 1738, 1991) A normal range for cats has not been previously established, however its similarity to that of the dog suggests that there is little difference in circulating ceruloplasmin levels between these two species. The normal ranges established during this study may be used to evaluate the health status of cats and dogs.

### Comparative Example 9

Canine ferritin : Assay validation and normal range for serum.

Ferritin plays an important role in the antioxidant defence system with the body. As a high affinity storage protein for iron, ferritin maintains iron in a safely bound form preventing the reactive ferrous ion from participating in Fenton reactions, which can lead to oxidative damage. In normal health ferritin, a species-specific protein, if found in the blood at concentrations that reflect body iron stores and in conjunctions with other parameters can be used to assess *in vivo* iron status. In order to determine circulating ferritin levels in the dog, an enzyme-linked immunoassay (ELISA) was developed, adapted from the method of Weeks, B.R. *et al* (1988) Am J Vet Res 49,1193) (1988) using monoclonal antibodies. Following the assay validation, a normal range for dogs was established.

In the range of 0-40 ng/ml the ferritin standards were linear (least squares regression analysis, r=0.997) and the recovery of purified ferritin added to canine sera was 97.7%. The intra-assay coefficient of variations derived from determining the ferritin concentration in two serum samples 12 time were 8.2% and 6.6%. The inter-assay coefficient of variations of two serum samples assayed 10 time on separate days were 16.6% and 16.2%.

Serum samples were obtained from 96 healthy dogs of mixed sex and five different breeds for ferritin determination in order to establish a normal range. Each sample was assayed either in triplicate or quadruplicate and the mean of these values was used as the ferritin concentration for that sample. The serum ferritin concentrations varied from 67.20 to 621.07 with a mean value of 371.62 ng/ml (SD 102.85 ng/ml). The data was normally distributed. These results demonstrate that serum ferritin can be determined with good repeatability and reproducibility for dogs. The values obtained will be used as a normal range for future studies and may provide a useful method of determining iron storage levels in dogs.

### Comparative Example 10

### Supplementation of dietary tocopherol increases canine plasma values outside the normal ranges

Vitamin E is the collective name for 8 naturally occurring molecules, 4 tocopherols and 4 tocotrienols. The biological activity of the various forms roughly correlates with their antioxidant activities with the order of relative peroxyl scavenging reactivities of α>β>γ>δ-tocopherol. Generally it is accepted that providing nutrients in excess of the requirement does not deliver any measurable benefit. The minimum requirement for vitamin E in adult dogs has been established as 2.5 IU α-tocopherol/400kcal diet (Nutrient Requirements of Dogs (1985) National Research Council (U.S.) National Academy Press Washington DC ISBN:0-309-03496-5). Prepared petfoods typically contain up to 10 times this amount but across the normal ranges of vitamin E in petfoods, plasma concentrations tend to be constant. In a study to evaluate the impact of supplementary vitamin E on the plasma response, 6 adult dogs, maintained on a nutritionally complete (see diet reference section) canned dog food (vitamin E content 8.2 mg/400kcal (8.2IU/400kcal), were offered a supplement equivalent to 100 IU vitamin E/day for 6 weeks. A control group of 6 dogs was maintained under the same conditions but received no dietary supplement. Plasma α-tocopherol was monitored during, and for 4 weeks following, the supplementation period.

The plasma α-tocopherol levels are presented in Table 6. The treatment group had a mean baseline plasma α-tocopherol level which was not significantly different from the control group (p=0.43). Following 2 weeks of supplementation the mean value of the treatment group had significantly increased compared to the control (p=0.002) and the baseline value (p<0.001). Measurements made on days 56 and 70 (days 14 and 28 post-supplementation) showed levels which remained significantly higher than baseline. These results clearly indicate that dietary vitamin E above the levels typically found in prepared petfoods can elevate plasma status in dogs and that this can be sustained for several weeks following supplementation. In order to saturate plasma it appears that levels greater than those currently found in prepared petfoods may need to be fed. Given the potent antioxidant capacity of vitamin E it may prudent to increase vitamin E status in dogs, in order to maximise the opportunity to prevent free-radical damage and the associated degenerative disease.

**Table 6. Plasma response to α-tocopherol supplementation in healthy dogs**

| | Baseline | Day 14 | Day 28 | Day 42 | Day 56 | Day 70 |
|---|---|---|---|---|---|---|
| Test | 15.9± 5.91◆ | 3.67±6.65 * | 32.6 ±8.39* | 35.4±7.07 * | 363±7.50 * | 28.2±6.8 7* |
| Control | 18.4±5.0 3*◆ | 22.2±7.29 | 19.9±4.00 * | 23.2±4.13 * | 30.5±7.9 3 | 23.3±3.7 1* |

| | | | | | | |
|---|---|---|---|---|---|---|
| * represents homogeneity (p>0.05) within the same group | | | | | | |
| ◆ represents homogeneity (p>0.05) between the 2 groups | | | | | | |

### Comparative Example 11

### Typical plasma vitamin E ranges in healthy dogs

Vitamin E is a collective name for eight different tocopherols and tocotrienols which share the same biological activity. Of the eight, α-tocopherol is biologically and chemically the most active form of vitamin E and 1mg α-tocopherol is equivalent to 1IU of vitamin E. Vitamin E is a potent antioxidant in the body, and it primarily resides in biological membranes where it protects membrane phospholipids from peroxidation damage. Vitamin E also inhibits oxidation of vitamin A and therefore also protects against vitamin A deficiency. The minimum requirement for vitamin E in adult dogs has been established as 2.5 IU α-tocopherol/400kcal diet (Nutrient Requirements of Dogs (1985) National Research Council (U.S.) National Academy Press Washington DC ISBN: 0-309-03496-5). However, there is a paucity of information as to normal vitamin E plasma values in healthy adult dogs and puppies. In order to determine typical baseline plasma α-tocopherol levels in healthy adult and puppy dogs, a series of studies were carried out in dogs fed a range of canned and dry nutritionally complete prepared petfoods (see diet reference section), Table 7. Diet was fed in accordance to body weight (110 kcal/kg^{0.75}). The individual plasma α-tocopherol levels ranged from 9 to 39.2 mg/l with a median value of 21.1 mg/l. There were no significant differences between the mean baseline α-tocopherol levels of the groups of dogs maintained on the different diets (p=0.24). These data indicate that typical α-tocopherol levels in puppies and adult dogs are similar and that, within the usual vitamin E levels found in prepared petfoods, plasma tocopherol levels exhibit similar ranges.

**Table 7. Diet α-tocopherol level (IU/400kcal) is shown with the corresponding means plasma α-tocopherol level (mg/l±SD).**

| Food format | α-Tocopherol content (IU/400kcal) | Plasma α-Tocopherol (mg/l±SD) | n |
|---|---|---|---|
| Canned | 9.77 | 21.15±6.4 | 20 |
| Dry | 6.31 | 22.72±6.62 | 6 |
| Dry | 13.76 | 25.33±5.12 | 10 |
| Dry | 18 | 21.56±4.88 | 23 |

### Comparative Example 12

Exercise in healthy adult dogs increases plasma TBARS-an indicator of oxidative stress.

Plasma TBARS (thiobarbituric acid reactive substances) measured by HPLC with pre-column derivatisation is a well-documented maker of lipid peroxidation *in vivo.* The aim of this present study was to survey the effects of a bout of exercise in dogs (*Canis familiaris*) upon this index of oxidative stress.

A panel of 14 dogs of mixed breed and age were maintained on a nutritionally complete commercial dry diet or three months prior to, and throughout the duration of this trial. The extent of lipid peroxidatation immediately prior to an following an acute 20 minute bout of paddock exercise was quantified by determining the malonaldehyde (MDA) formed as TBARS. This analysis was measured according to the method described by Bird, R.P. & Draper, H.H. (1984), Methods in Enzymology. 105:299-305 (1984). The results revealed a significant increase (22%) in plasma TBARS (Paired TTest p<0.05) following exercise (0.74 µM ± 0.2 pre-, 0.92 µM ± 0.2 post exercise). In order to audit for increases occurring as a direct result of concentrated blood volumes post-exercise, PCV (packed cell volumes) and plasma albumin were measured. Results did not reveal a significant difference pre- and post-exercise (p>0.05) and therefore the observations from this study suggest that augmented lipid peroxidation *in vivo* occurs as a direct result of exercise-induced oxidative stress. Literature proposes that the specific site of oxidative damage is the cellular membrane, where peroxyl radicals (RO₂) proliferate in conditions of high oxidative stress. This observation infers a potential role for dietary lipid-phase antioxidant intervention in healthy adult dogs.
Other examples of the antioxidative status of animals and the effects of the inventive antioxidant cocktail on animals are described below.

### Comparative Example 13

### Dietary Carotenoid Absorption in the Domestic Cat.

It is found that antioxidants, such as beta-carotene and lycopene, incorporated into commercial cat food will result in a significant increase in the absorption of carotenoids in cats, despite their metabolic carnivorous adaptation. In this study three canned cat diets were manufactured using the same batch of raw ingredients with an identical base recipe. The control diet, Diet A, had a metabolisable energy (ME) content of 3.39 MJ/kg with a beta-carotene and lycopene content of >0.1 mg/1.67 MJ ME. Diet B was supplemented with red palm oil (3.70 MJ ME/kg) with a beta-carotene content of 0.36 mg/1.67 MJ ME, and a lycopene content of >0.1 MG/1.67 MJ ME and Diet C was supplemented with tomato pumice (3.54 MJ ME/kg) with a beta-carotene content of >0.1 MG/1.67 MJ ME and a lycopene content of 0.9 MG/1.67 MJ ME.

Two groups of five healthy cats each were selected for the study. The cats of Group 1 had a mean age of 8.72 years (SD2.1 years) and included 3 males and 2 females. The Group 2 cats had a mean age of 7.4 years (SD 1.7 years) and included 2 males and 3 females. All of the study cats were previously maintained on a variety of commercial, nutritionally complete, prepared cat food and then were maintained on Diet A for a period of seven days to allow acclamation to the diet. On day eight of the study, a baseline plasma carotenoid level was determined. Subsequently, Group 1 cats were fed Diet B and Group 2 cats were fed Diet C, for a period of five days. On day 13 of the study, a plasma sampling was repeated in the same manner as for the baseline samples. The analyses were carried out by High Performance Liquid Chromatography.

The beta-carotene concentration of the Group 1 cats on Diet B, increased significantly (mean 17.62 ng/mL, SD 2.50 ng/mL) above the baseline values (mean 6.35 ng/mL. SD 3.23 ng/mL). In this group, the plasma lycopehe concentrations remained below the limit of detection. In the Group 2 cats, on Diet C, there was no significant change in the plasma beta-carotene concentrations of the baseline levels (mean 5.30 ng/mL, SD 5.78 ng/mL) and the post-feeding levels (mean 6.61 ng/mL, SD 2.83ng/mL). However, Group 2 cats showed a significant increase in plasma lycopene concentrations from a baseline level of 0 ng/mL to a post-feeding level of mean 14.6 ng/mL, SD 7.25 ng/mL.

Thus, this study demonstrates that natural sources of antioxidants, such as beta-carotene and lycopene, incorporated into commercial cat food will result in a significant increase in the absorption of carotenoids in cats, despite the metabolic carnivorous adaption. This increase in circulating antioxidants will provide a beneficial effect to the antioxidative status of cats. This increased absorption of carotenoids in cats has not heretofore been seen at such low dietary concentrations.

### Comparative Example 14

Maternal milk may be insufficient to promote a maximal antioxidant status in the developing kitten.

With respect to immune function, the effect of antioxidants is equally applicable to animals who have a compromised immune function due to age, e.g. growing animals, as well as those experiencing immunological challenges. The results of the following study show that maternal milk may be insufficient to promote a maximal antioxidant status in the developing kitten, which in turn may contribute to the increased susceptibility of kittens to oxidative stress. Thus, it can be found that the administration of an antioxidant cocktail as a dietary supplement for kittens will likewise show an improvement in antibody response time as is found in puppies receiving the antioxidant cocktail supplement.

The ability of mammals to resist oxidative insult depends on both their endogenous antioxidant defense systems and the contribution to overall antioxidant status provided by diet. One way to measure an animal's antioxidant status is to evaluate its total plasma antioxidant capacity (TPAO). A colormetric assay kit manufactured by Randox Laboratories Ltd. has been validated for use in cats to assay the normal range of TPAO. Antioxidants present in blood samples will cause suppression of the color production in the assay proportional to their blood concentration.

This study was conducted to determine the normal range of TPAO in healthy kittens and to determine whether there is any relationship between age and TPAO. Plasma samples were obtained from 16 health kittens (8 males, 8 females) at 14, 35 and 60 days of age. The samples were assayed using the method as described above and the results were compared using ANOVA. The results are illustrated in table 7 below:

**Table 7.**

| Age of Kittens | TPAO |
|---|---|
| 14 days | 0.694 ± 0.069 |
| 35 days | 0.853 ± 0.083 |
| 60 days | 1.030 ± 0.113 |

From the results, it can be seen that the TPAO status of suckling kittens is at the bottom end of the normal adult range, only achieving adult levels between 35 and 60 days. At 14 days after birth, kittens are completely dependent upon maternal milk to provide their nutritional requirements. At 35 days of age the kittens are far less dependent on maternal milk and obtain the majority of their nutritional intake from sold foods, while at 60 days they are fully weaned. These results suggest that maternal milk is insufficient to promote maximal antioxidant status in the developing kitten, which in turn contributes to the increased susceptibility of kittens to oxidative stress.

### Example 15

### The antioxidant fortified diet increases the antioxidant capacity of cats.

A group of 46 healthy domestic short haired cats were randomly stratified into 2 groups of 23 cats dependent upon age and sex. Group A were maintained on a control base diet (a complete wet diet according to the reference section) and Group B on a wet diet supplemented with an antioxidant cocktail for a period of 30 weeks.

The cocktail was:

| | |
|---|---|
| Vitamin E | 50IU/400kcal diet |
| Vitamin C | 20mg/400kcal diet |
| Beta-carotene | 0.5-1mg/400kcal diet |
| Lutein | 0.5mg/400kcal diet |
| Taurine | 200mg/400kcal diet |
| Lycopene | 1mg/400mg kcal diet |

Fasting samples were obtained from all cats and assessed for antioxidant capacity using the ferric reducing antioxidant power (FRAP) and the ferric reducing antioxidant power and ascorbic acid concentration (FRASC) assays.

The antioxidant capacity was significantly increased (p<0.05) in Group B compared to Group A with respect to both FRAP and FRASC.

| GROUP | MEAN FRAP ± SD | MEAN FRASC ± SD |
|---|---|---|
| A | 260.45 ± 55.59 | 28.05 ± 7.93 |
| B | 297.63 ± 57.18 | 36.33 ± 10.69 |

Hence, the antioxidant capacity was increased in cats fed the antioxidant fortified diet, which confers an increased ability to mitigate the deleterious effects associated with oxidative insult.

### Example 16

### Influence of the antioxidant supplemented diet on the immunological status of cats

### EXPERIMENTAL DESIGN:

48 normal healthy cats were fed a control diet (complete diet as per the reference section) for six weeks after which baseline measurements were taken. Cats were then allocated to either control or treatment age -matched groups and fed the supplemented diet described in Example 15. At week eight the animals were sampled in order to determine serum immunoglobulin concentrations. At week twelve immune parameters were measured and the cats were immunised (using a standard combined vaccine against Feline Panleucopenia, Feline Calicivirus and Feline Herpesvirus). At week eighteen final measurements were made post immunisation.

### METHODS USED:-

### Assessment of peripheral blood mononuclear cellular (PBMC) proliferative response by mitogen induced lyniphocyte transformation assay (MILT)

Peripheral blood mononuclear cells were isolated from heparinised blood by density gradient centrifugation on Histopaque 1077(Sigma). The cells were washed twice with phosphate buffered saline (PBS) and once with RPMI-1640 (Dutch modification) supplemented with 10 per cent heat inactivated fetal calf serum, 1 per cent penicillin/streptomycin and 2 per cent sodium pyruvate. Cell viability was assessed by the trypan blue exclusion test (Sigma).

Cells were cultured in triplicate at 1 x 10⁵ per well in 96 well flat bottomed microtitre plates at 37°C, with phytohaemagglutinin (PHA) (5 µg/ml)(Murex), concanavalin A (Con A) (7.5 µg/ml) and pokeweed mitogen (PWM)(1 µg/ml)(Sigma) for 96 hrs.

Proliferation was measured by [³H]-thymidine incorporation in counts per minute (CPM) (0.5 µCi/well) during the final 18 hrs of culture.

### Analysis of lymphocyte subsets by flow cytometry

CD4 and CD8 positive cells are the most well characterised lymphocyte subsets in feline immunology and an adequate repertoire of these cells is indicative of a healthy immune system. The assay was performed using both purified lymphocytes and whole blood and a selection of various monoclonal antibodies (Mabs).

### RESULTS

### Assessment of PBMC proliferative response by mitogen induced lymphocyte transformation assay (MILT)

Table 8 shows the response of PBMC to the mitogens PHA, Con A and PWM prior to and post immune challenge Mitogen induced lymphocyte transformation assay (MILT) data showed no significant changes in proliferative response for either control or treatment groups. When an analysis of stimulation indices was undertaken there was a significant decrease in the treatment group in both the PHA stimulation index (S I) (p<0.05) and the Con A index (p<0.001) from pre to post-immunisation. There was no significant difference in the SI of the control group. The Pokeweed SI increased significantly from baseline to pre-immunisation in both groups (p<0.05) and decreased significantly in the treatment group post immunisation (p<0.01).

**Table 8.**

| The response of PBMC to the mitogens PHA, Con A and PWM prior to and post immune challenge. | | | | | | |
|---|---|---|---|---|---|---|
| [³H] thymidine incorporation (counts per min) CPM x 10⁻³ MEAN ± SEM Stimulation index (S.I.) MEAN ± SEM | | | | | | |
| | Baseline | | Pre-immunisation | | Post-immunisation | |
| | standard^{a} | lara plus^{b} | standard | lara plus | standard | lara plus |
| | (*n* =22) | *(n* = 23) | *(n* =22) | (*n* = 23) | *(n* =22) | (*n* = 23) |
| | | | | | | |
| Unstimulated | 20 ± 4 | 21.6 ± 4 | 27.2 ± 3.6 | 18.2 ± 3 | 30.9 ±3.9 | 25 ±2.6 |
| | | | | | | |
| PHA | 38.8±4 | 41.5 ± 6 | 33.7±3.9 | 28.5±3.2 | 38.8±4 | 33.3±3 |
| *S.I.* | *2.01* ± *0.2* | *2.69* ± *0.5* | *1.38* ± *0.1* | *2.2* ± *0.25* | *1.8* ± *0.58* | *1.49* ± *0.14** |
| | | | | | | |
| Con A | 29 ± 3 | 32.4 ± 4.5 | 31.4 ± 3.5 | 38 ± 5.9 | 37.9 ±4 | 33 ± 3.6 |
| *S.I.* | *1.55 ±* 0.1 | *1.85 ±* 0.2 | *1.38 ±* 0.1 | *2.3 ± 0.1* | *1.81 ± 0.5* | *1.46 ± 0.15**** |
| | | | | | | |
| PWM | 21.5 ± 2.5 | 22 ± 4 | 37 ± 4.2 | 29.7 ± 3 3 | 37.8 ± 4 | 29.7 ± 3.3 |
| *S.l.* | *1.1* ± *0.1* | *1.25* ± *0.15* | *1.5* ± *0.2** | *2.52* ± *0.4* * | *2.03* ± *0.7* | *1.26* ± 0.1** |

| | | | | | | |
|---|---|---|---|---|---|---|
| ^{a}control group, standard diet * P < 0.05 ; ** P < 0.01 ; | | | | | | |
| ^{b} treatment group, test diet *** P < 0.001 | | | | | | |

### Analysis of lymphocyte subsets by flow cytometry

Table 9 shows T-cell relative subset counts and CD4+: CD8+ ratio pre and post immunisation. When CD4 and CD8 T-cell subsets were analysed there was a significant increase in percentage of CD4 positive cells (p<0.05) in both groups and a significant increase in CD8 positive cells in both the control group (p< 0.05) and test group (p<0.001) post immunisation.

When the CD4 +: CD8 + ratio of lymphocytes was examined it was found to be decreased significantly in the control group (p<0.001) while remaining constant in the treatment group post immune challenge. When examining age relationships there was a trend towards a decreasing CD4+: CD8+ ratio with increasing age in the control group prior to immunisation (r = -0.483, p<0.05).

**Table 9**

| T-cell relative subset counts and CD4+: CD8+ ratio pre and post immunisation, MEAN ± SEM | | | | |
|---|---|---|---|---|
| | Pre immunisation | | Post immunisation | |
| | standard ^{a} | lara plus ^{b} | standard | lara plus |
| | *(n* = 21) | *(n* = *23)* | *(n* = *21)* | *(n* = *23)* |
| CD4 positive | | | | |
| percentage | 22.6 ± 1.1 | 20.9 ± 0.7 | 25.1 ± 1.6 * | 24.5 ± 1.3* |
| | | | | |
| CD8 positive | | | | |
| percentage | 17.2 ± 0.1 | 15.4 ± 1.3 | 22.3 ± 1.2 | * 19.6 ± 1.6** |
| | | | | |
| CD4:CD8 | | | | |
| ratio | 1.42 ± 0.1 | 1.57 ± 0.1 | 1.17 ± 0.1** | 1.43 ± 0.1 |

| | | | | |
|---|---|---|---|---|
| ^{a} control group, standard diet * P < 0.05 ; ;** P < 0.001 | | | | |
| ^{b}treatment group, test diet | | | | |

The observed difference in SI of PWM stimulated cells from cats fed the supplemented diet (Table 8) suggests that there is a beneficial upregulation of CD2, an activation marker of T-cells.

The results on Table 9 show clearly the beneficial effects of the supplemented diet on the CD4:CD8 ratios of cats post-vaccination. The CD4:CD8 ratio in the supplemented cats was maintained post vaccination compared to the control group. This maintenance is mainly due to an increase in CD4.

These facts show beneficial effects of the supplement upon the immune response of cats.

### Example 17

Effects of an Antioxidant Cocktail on Specific Antibody Responses of Young Dogs
- Litters of Labrador and Greyhound Puppies were separated into two age and sex - matched groups.
- One group of each breed had their standard diet (complete, as per the reference section) supplemented with a cocktail (details given below), the other two groups (one of each breed), remained on an unsupplemented diet.

### Antioxidant Cocktail-

| | |
|---|---|
| alpha-tocopherol | 50mg /400kcal |
| ascorbate | 20mg /400kcal dry (40mg if wet) |
| beta-carotene | 0.5mg /400kcal |
| lutein | 0.5mg /400kcal |
| taurine | 200mg /400kcal dry (500mg if wet) |

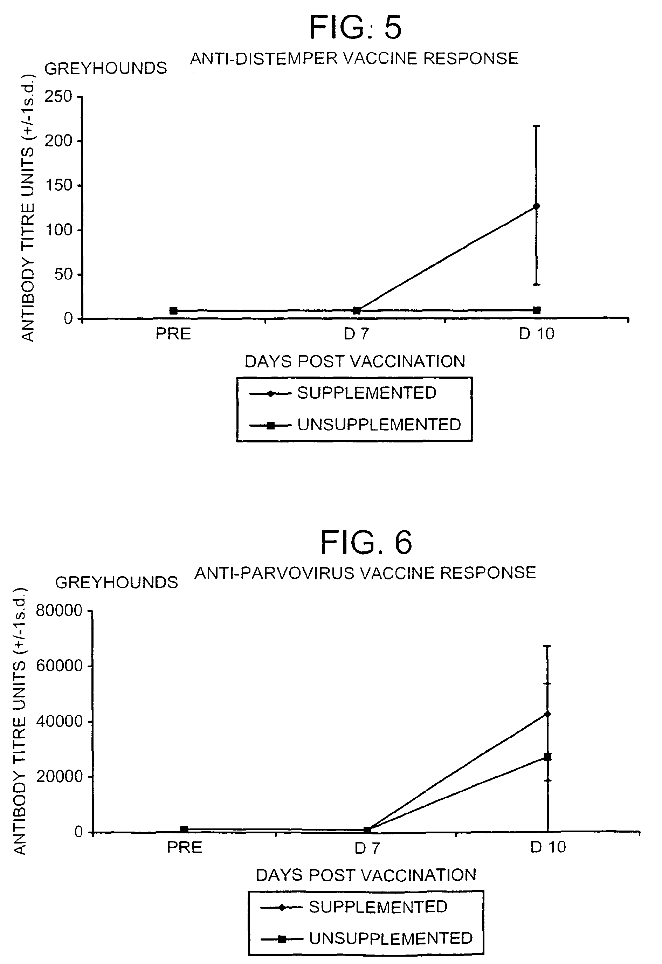

- Supplement was administered for up to a maximum of four weeks prior to vaccination.
- All of the puppies were vaccinated according to routine vaccination procedures (vaccines included Parvo-virus and Distemper).
- Antibody levels to vaccine antigens were measured for all puppies.
- Some of these results are shown on Figures 4, 5 and 6.
- These results clearly indicate that puppies receiving a supplement of the antioxidant cocktail will mount a faster response to specific antigens such as are introduced via a vaccine or which may be introduced through exposure to an infectious agent.
- These results show that the antioxidant cocktail has a highly beneficial effect on the immune response of young animals.

### Example 18

Beneficial Effects of Antioxidant Cocktail on the Maintenance of a Vaccine Response in Adult and Senior Dogs
- Two groups of dogs were age, sex and breed matched.
- Both groups were further matched in accordance to when they had previously been vaccinated (prior to the start of the study).
- One group was fed a diet supplemented with an antioxidant cocktail (details given below), the other group remained an unsupplemented control.

**Table 10**

| Antioxidant Content of Diet | Supplemented Test Diet | Control Diet |
|---|---|---|
| *Vitamin E* | 52.41 IU/400 kcal | 4.81 IU/400 kcal |
| *Vitamin C* | 65.9 mg/400 kcal | 2.48 mg/400 kcal |
| *Taurine* | 0.16% | 0.054% |
| *Carotenoids* | | |
| *-Cis Beta-carotene* | 11.07 ug/400 kcal | <10.96 ug/400 kcal |
| *-Trans Beta-carotene* | 33.21 ug/400 kcal | 21.91 ug/400 kcal |
| *-Trans Alpha-carotene* | <11.07 ug/400 kcal | 10.96 ug/400 kcal |
| *-Cis Alpha-carotene* | <11.07 ug/400 kcal | <10.96 ug/400 kcal |
| *-Lutein* | 0.996 mg/400 kcal | 0.877 mg/400 kcal |
| *-Lycopene* | <11.07 mg/400 kcal | <10.96 ug/400 kcal |
| *-Xeaxanthian* | 1.22 mg/400 kcal | 1.32 mg/400 kcal |

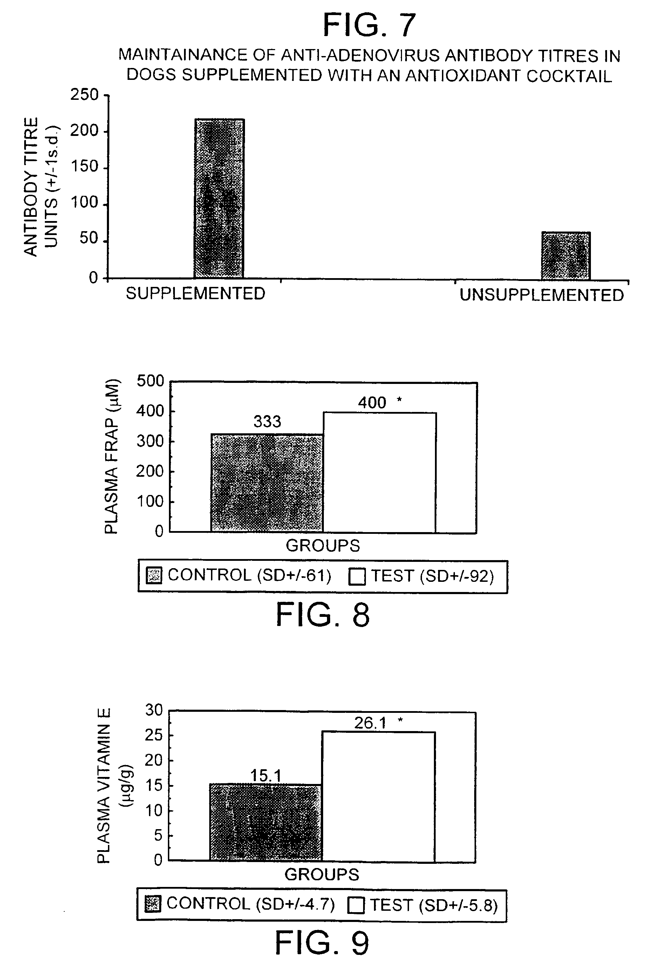

- After a period of six months on the supplemented diet the dogs had their circulating anti-adenovirus antibody titre measured. Results are shown on the graph in Figure 7.
- These results show that animals fed a diet containing an antioxidant cocktail are better able to maintain vaccine induced antibodies over time than are unsupplemented dogs.

### Example 19

The antioxidant status and oxidative damage in dogs fed a canine dry diet containing an antioxidant cocktail after 8 weeks.

### Summary:

- This report contains results (antioxidant status and oxidative damage) from dogs fed a canine dry diet containing an antioxidant cocktail for 8 weeks. Some of the results were influenced by diet and age.
- Plasma FRAP and vitamin E levels in the Antioxidant-fed group were significantly higher than in the Control-fed group.
- Plasma vitamin E levels in both the Young Adult and Senior dogs fed the Antioxidant diet were significantly higher than in their respective Control groups.

### MATERIALS & METHODS

### Animals

### 1) Type of animals

40 young adult (0.8 to 3.3 years old) and senior (6.5 to 12.5 years), pure breeds (Labradors, Beagles, West Highland white terrier, Newfoundland, & Golden Retriver), and mixed sex dogs (intact, neutered/spayed) were included in the study.

### 2) Housing

Dogs were grouped in pairs and had access to the outside environment from their pens. All dogs had access to indoors during day and night times. Dogs from the same treatment group were housed together to prevent cross contamination due to coprophagia (fecal consumption). Temperature was controlled at 22°C with natural light cycle. Dogs were allowed to follow their daily regular exercise routine.

### 3) Feeding Schedule

All dogs were on a complete and balanced canine dry diet for one week prior to the start of the study. After the one week of adaptation, two-thirds of the dogs remained on the base diet as the control group, while the remaining third was switched over to the test diet. The amount of food fed to each dog was based on the ME equation (110 x BW ^ 0.75 Kcal). The amount of food fed to each dog was adjusted accordingly to maintain bodyweight. All dogs had access to fresh water at all times.

### Test Substance

The test diet contained the following ingredients that were not added to the control diet.

| Ingredients | % Added to diet |
|---|---|
| Tomato Pomace | 5 |
| Vitamin C 35% | 0.18 |
| Taurine | 0.14 |
| Vitamm E 50% | 0.12 |
| Marigold Meal | 0.04 |

### Trial Design

The study was a longitudinal test design. All dogs were randomly assigned based on breed, sex, age, body weight and health status.

| Groups | n's per group | Age (yrs) | Diets | Test Period |
|---|---|---|---|---|
| Control (A,B,E,F) | 26 | 5.5 ± 4.3 | Control Diet (w/o antioxidant cocktail) | 8 weeks |
| Test (C, G) | 14 | 5.9 ± 4.4 | Antioxidant Cocktail Diet | 8 weeks |

Same dogs as the above table, however, they were broken down by age.

| Groups | n's per group | Avg age w/in each group (years ± SD) | Diets | Test Period |
|---|---|---|---|---|
| Young Adult Control (A,B) | 12 | 1.2 ± 0.8 | Control Diet | 8 weeks |
| Young Adult Test (C) | 6 | 1.0 ± 0.1 | Antioxidant Cocktail Diet | 8 weeks |
| Senior Control (E,F) | 14 | 9.3 ± 2.0 | Control Diet | 8 weeks |
| Senior Test (G) | 8 | 9.5 ± 1.5 | Antioxidant Cocktail Diet | 8 weeks |

*Blood and saliva biomarkers:* Bloods were collected from each dog to measure the following parameters at 8 weeks after the treatment.

| Antioxidants/Damage | Description |
|---|---|
| Plasma vitamin E | Fat soluble antioxidant |
| Plasma FRAP/FRASC | A measure of "Antioxidant Power" in plasma & ascorbic acid level |

### Statistics

Analyses were determined for all dogs for all parameters by unequal n's Tukey's post hoc test for statistical significance between treatment at p < 0.05. Data are expressed as group means ± SD.

Figure 8: The measurement of ferric reducing ability of plasma (FRAP) that measures the "antioxidant capacity" in dogs fed a canine dry diet containing an antioxidant cocktail for a period of 8 weeks. (Control, n=26; Test, n=14). *Represents significant differences from the Control group, p <0.05.
- The measurement of plasma "antioxidant capacity" in the Test group was significantly higher than in the Control group after 8 weeks of treatment.
   Figure 9: The measurement of plasma vitamin E levels in dogs fed a canine dry diet containing an antioxidant cocktail for a period of 8 weeks. (Control, n=26; Test, n=14). *Represents significant differences from the Control group, p<0.05.
- Plasma vitamin E level in the Test group was significantly higher than in the Control group after 8 weeks of treatment.

### Reference Diet Section

Nutritionally complete diet

A complete diet for foodstuff, especially a nutritionally complete petfood (or diet) is a diet which meets all the nutritional requirements of the individual animal's lifestyle and lifestage.

The diet or foodstuff can be made according to any method known in the art, such as in Waltham Book of Dog and Cat Nutrition, Ed. ATB Edney, Chapter by A. Rainsbird, entitled "A Balanced Diet" in pages 57 to 74, Pergoren Press Oxford.

The following shows a composition of a complete balanced diet according to the Examples.

| Ingredient | Inclusion |
|---|---|
| Rice | 24.9% |
| Whole corn | 18.8% |
| Whole grain wheat | 12.2% |
| Chicken by-product meat | 18.7% |
| Corn gluten meal | 9.5% |
| Brewers yeast | 1.7% |
| Dried egg | 0.8% |
| Non-iodinised salt | 0.7% |
| Vitamin premix | 3.4% |
| Sunflower oil | 0.5% |
| Beef tallow | 4.9% |
| Poultry viscera | 4.4% |

| | |
|---|---|
| Analytical profile - moisture 8.2%, protein 26.4%, fat 10.4%, ash 7.1%, fibre 2.2% (the remainder being made up of nitrogen-free extract (mainly carbohydrate)). | |

## Claims

1. A dog or cat foodstuff, diet or supplement which provides a concentration of vitamin E at a level of 25IU/400kcal or above, a concentration of vitamin C of 10mg/400kcal or above and a concentration of taurine of 80mg/400kcal or above, for use in strengthening the immune response.

2. A foodstuff, diet or supplement, as claimed in claim 1, which further provides a carotenoid of from 0.01mg/400kcal or above,

3. A foodstuff, diet or supplement, as claims in claim 2, wherein the carotenoid is one or more of beta-carotene, alpha-carotene, lycopene, lutein, zeaxanthin or antaxanthin.

4. A foodstuff, diet or supplement, as claimed in any one of claims 1-3, for use in the prevention or treatment of any disorder which has a component of oxidative stress.

5. Use of vitamin E, vitamin C and taurine, in the manufacture of dog or cat foodstuff, diet or supplement for strengthening the immune response of cat or dog.

6. Use, as claimed in claim 5, wherein the vitamin E is present at a concentration of 25IU/400kcal or above.

7. Use, as claimed in claim 5 or claim 6, wherein the vitamin C is present at a concentration of 80mg/400kcal or above.

8. Use, as claimed in any one of claims 5 to 8, wherein the foodstuff, diet or supplement further comprises a carotenoid.

9. Use , as claimed in claim 9, wherein the carotenoid is one or more of beta-carotene, lycopene, lutein, zeaxanthin or astaxanthin

10. Use, as claimed in any one of claims 5 to 10 for treating or assisting a cat or dog in response to the immune challenge or vaccination.

11. A method of making a foodstuff, diet or supplement, as claimed in anyone of claims 1-4, the method comprising mixing together the ingredients of the foodstuff.

## Patentansprüche

1. Futtermittel, Ernährung oder Ergänzungsstoff für Hund oder Katze, das/die/der eine Konzentration an Vitamin E bei einem Gehalt von 25 IU/400 kcal oder mehr, eine Konzentration an Vitamin C von 10 mg/400 kcal oder mehr und eine Konzentration an Taurin von 80 mg/400 kcal oder mehr bereitstellt, zur Verwendung bei der Stärkung der Immunreaktion.

2. Futtermittel, Ernährung oder Ergänzungsstoff nach Anspruch 1, das/die/der weiter ein Carotinoid mit von 0,01 mg/400 kcal oder mehr bereitstellt.

3. Futtermittel, Ernährung oder Ergänzungsstoff nach Anspruch 2, **dadurch gekennzeichnet, daß** das Carotinoid eines oder mehrere von beta-Carotin, alpha-Carotin, Lycopin, Lutein, Zeaxanthin oder Astaxanthin ist.

4. Futtermittel, Ernährung oder Ergänzungsstoff nach einem der Ansprüche 1 bis 3 zur Verwendung bei der Prävention oder Behandlung jeder Störung, die eine Komponente oxidativen Streß aufweist.

5. Verwendung von Vitamin E, Vitamin C und Taurin bei der Herstellung von Futtermittel, Ernährung oder Ergänzungsstoff für Hund oder Katze zur Stärkung der Immunreaktion von Katze oder Hund.

6. Verwendung nach Anspruch 5, **dadurch gekennzeichnet, daß** das Vitamin E in einer Konzentration von 25 IU/400 kcal oder mehr vorliegt.

7. Verwendung nach Anspruch 5 oder 6, **dadurch gekennzeichnet, daß** das Vitamin C in einer Konzentration von 80 mg/400 kcal oder mehr vorliegt.

8. Verwendung nach einem der Ansprüche 5 bis 8, **dadurch gekennzeichnet, daß** das Futtermittel, die Ernährung oder der Ergänzungsstoff weiter ein Carotinoid umfaßt.

9. Verwendung nach Anspruch 9, **dadurch gekennzeichnet, daß** das Carotinoid eines oder mehrere von beta-Carotin, Lycopin, Lutein, Zeaxanthin oder Astaxanthin ist.

10. Verwendung nach einem der Ansprüche 5 bis 10 zur Behandlung oder Unterstützung einer Katze oder eines Hundes bei der Reaktion auf die Immunherausforderung oder Impfung.

11. Verfahren zur Herstellung eines Futtermittels, einer Ernährung oder eines Ergänzungsstoffes nach einem der Ansprüche 1 bis 4, wobei das Verfahren das Miteinandervermischen der Inhaltsstoffe des Futtermittels umfaßt.

## Revendications

1. Aliment, nourriture ou supplément alimentaire pour chien ou chat qui fournit une concentration en vitamine E d'un niveau de 25 UI/400 kcal ou plus, une concentration en vitamine C de 10 mg/400 kcal ou plus et une concentration en taurine de 80 mg/400 kcal ou plus, destiné à être utilisé pour le renforcement de la réponse immunitaire.

2. Aliment, nourriture ou supplément alimentaire selon la revendication 1, qui fournit de plus un caroténoïde à raison de 0,01 mg/400 kcal ou plus.

3. Aliment, nourriture ou supplément alimentaire selon la revendication 2, dans lequel le caroténoïde est un ou plusieurs caroténoïdes choisis dans le groupe comprenant le bêta-carotène, l'alpha-carotène, le lycopène, la lutéine, la zéaxanthine ou l'astaxanthine.

4. Aliment, nourriture ou supplément alimentaire selon l'une quelconque des revendications 1 à 3, utile pour la prévention ou le traitement d'un quelconque trouble qui présente une composante de stress oxydant.

5. Utilisation de vitamine E, de vitamine C et de taurine dans la fabrication d'un aliment, d'une nourriture ou d'un supplément alimentaire pour chien ou chat pour le renforcement de la réponse immunitaire d'un chat ou d'un chien.

6. Utilisation selon la revendication 5, dans laquelle la vitamine E est présente à une concentration de 25 UI/400 kcal ou plus.

7. Utilisation selon la revendication 5 ou la revendication 6, dans laquelle la vitamine C est présente à une concentration de 80 mg/400 kcal ou plus.

8. Utilisation selon l'une quelconque des revendications 5 à 8, dans laquelle l'aliment, la nourriture ou le supplément alimentaire comprend de plus un caroténoïde.

9. Utilisation selon la revendication 8, dans lequel le caroténoïde est un ou plusieurs caroténoïdes choisis dans le groupe comprenant le bêta-carotène, l'alpha-carotène, le lycopène, la lutéine, la zéaxanthine ou l'astaxanthine.

10. Utilisation selon l'une quelconque des revendications 5 à 9 pour traiter ou aider un chat ou un chien en réponse à une épreuve immunitaire ou une vaccination.

11. Procédé de préparation d'un aliment, d'une nourriture ou d'un supplément alimentaire selon l'une quelconque des revendications 1 à 4, le procédé comprenant le mélange entre eux des ingrédients de l'aliment.
